# EUROPEAN PATENT SPECIFICATION

(11) **EP 3 941 465 B1**
(45) Date of publication and mention of the grant of the patent: **17.09.2025**
(21) Application number: 20712563.4
(22) Date of filing: 20.03.2020
(51) Int. Cl.: A61K 31/4245, A61K 9/00, A61P 25/00

(54) **METHODS OF TREATING ATTENTION DEFICIT HYPERACTIVITY DISORDER USING KDM1A INHIBITORS SUCH AS THE COMPOUND VAFIDEMSTAT**
METHODEN ZUR BEHANDLUNG VON AUFMERKSAMKEITSDEFIZIT/HYPERAKTIVITÄTSSTÖRUNGEN UNTER VERWENDUNG VON KDM1A INHIBITOREN WIE DER VERBINDUNG VAFIDEMSTAT
METHODES POUR LE TRAITEMENT DU TROUBLE DU DÉFICIT DE L'ATTENTION ET HYPERACTIVITÉ EN UTILISANT UN INHIBITEUR DE KDM1A TEL QUE LE COMPOSE VAFIDEMSTAT

(30) Priority: 20.03.2019 EP 19382198
(43) Date of publication of application: 26.01.2022
(73) Proprietor: Oryzon Genomics, S.A., 28014 Madrid (ES)
(72) Inventor: BUESA ARJOL, Carlos Manuel, 08940 Cornellà de Llobregat (ES); BULLOCK, Roger Alan, 08940 Cornellà de Llobregat (ES); RAMOS QUIROGA, José Antonio, 08035 Barcelona (ES)
(74) Representative: Vossius & Partner Patentanwälte Rechtsanwälte mbB
(86) International application number: PCT/EP2020/057800
(87) International publication number: WO 2020/188089

(56) References cited:
- WO-A1-2019/025588
- LIANG-JEN WANG ET AL: "Clinical symptoms and performance on the continuous performance test in children with attention deficit hyperactivity disorder between subtypes: a natural follow-up study for 6 months", BMC PSYCHIATRY, BIOMED CENTRAL, LONDON, GB, vol. 11, no. 1, 19 April 2011 (2011-04-19), pages 65, XP021099385, ISSN: 1471-244X, DOI: 10.1186/1471-244X-11-65
- WELLER E B ET AL: "AGGRESSIVE BEHAVIOR IN PATIENTS WITH ATTENTION-DEFICIT/HYPERACTIVITY DISORDER, CONDUCT DISORDER, AND PERVASIVE DEVELOPMENTAL DISORDERS", JOURNAL OF CLINICAL PSYCHIATRY, PHYSICIANS POSTGRADUATE PRESS, INC, US, vol. 60, no. SUPPL. 15, 1 January 1999 (1999-01-01), pages 5 - 11, XP008019349, ISSN: 0160-6689
- YANG ET AL: "Cortisol is inversely correlated with aggression for those boys with attention deficit hyperactivity disorder who retain their reactivity to stress", PSYCHIATRY RESEARCH, ELSEVIER IRELAND LTD, IE, vol. 153, no. 1, 11 September 2007 (2007-09-11), pages 55 - 60, XP022241323, ISSN: 0165-1781, DOI: 10.1016/J.PSYCHRES.2006.04.001

## Description

### FIELD

The present invention relates to methods for treating attention deficit hyperactivity disorder.

### BACKGROUND

Attention deficit hyperactivity disorder (ADHD) is a neural development disorder that is characterized by a persistent pattern of difficulty paying attention, hyperactivity and impulsiveness. It is considered to be a chronic disorder that commences in infancy and persists into adult age in more than 50% of cases. Adult ADHD has a prevalence about 2.5-4% of the general adult population. ADHD is associated in adults as well as children with a general pattern of problems in academic performance and social, family and work-related adaptation, giving rise to high economic and healthcare costs.

Thus, there is a strong and unmet medical need for new and/or improved drugs for treating ADHD, including adult ADHD, particularly drugs that act via novel mechanisms of action and treat the core features of ADHD, and/or with a more favorable side effect profile than current therapies. The present invention addresses these and other needs.

WO 2019/025588 discloses the usefulness of a KDM1A inhibitor, in particular vafidemstat, or of a pharmaceutical composition comprising said KDM1A inhibitor for use in the treatment of behaviour alteration, such as aggressiveness. Said aggressiveness can be associated with adult attention deficit hyperactivity disorder (A-ADHD).

Liang-Jen Wang et al. ("Clinical symptoms and performance on the continuous performance test in children with attention deficit hyperactivity disorder between subtypes: a natural follow-up study for 6 months", BMC Psychiatry (2011), vol. 11, no. 1, 65) disclose inter alia results from 6 months of real-world clinical treatment in relation to differences in the ADHD symptom composite scores between aggressive and non-aggressive ADHD patients undergoing treatment with methylphenidate (MPH).

Weller et al. (Aggressive Behavior in Patients with Attention-Deficit/Hyperactivity Disorder, Conduct Disorder, and Pervasive Developmental Disorders", Journal of Clinical Psychiatry (1999), vol. 60, Suppl. 15, 5-11) disclose inter alia that children who have aggressive behavior with ADHD differ from those who have AHDH without aggression.

Yang et al. ("Cortisol is inversely correlated with aggression for those boys with attention deficit hyperactivity disorder who retain their reactivity to stress", PSYCHIATRY RESEARCH (2007), vol. 153, no. 1, 55-60) examined the relationship between the cortisol response to stress and aggression in patients with attention deficit hyperactivity disorder (ADHD).

### SUMMARY OF THE INVENTION

The invention provides novel methods for treating attention deficit hyperactivity disorder by treating one or more non-aggressive symptoms of ADHD using a KDM1A inhibitor.

Thus, the present invention provides a KDM1A inhibitor for use in the treatment of attention deficit hyperactivity disorder, as defined in the claims.

The KDM1A inhibitor of the present invention is vafidemstat or a pharmaceutically acceptable salt or solvate thereof.

In some embodiments, the attention deficit hyperactivity disorder is adult attention deficit hyperactivity disorder.

The invention is defined in the claims. Any subject-matter beyond the scope of the claims is provided for comparative or reference purposes only.

Any reference to a method of treatment of the human or animal body by therapy using a certain compound or composition is to be understood as a reference to said compound or composition for use in said therapy.

### BRIEF DESCRIPTION OF THE DRAWINGS

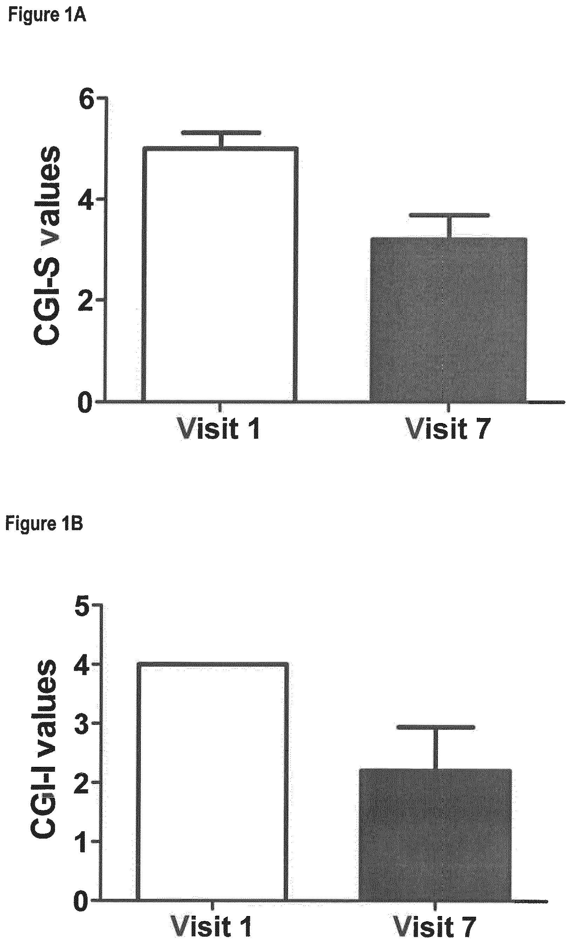

Reference Figure 1 shows the effect of treatment with the KDM1A inhibitor vafidemstat (as defined herein and in Example 1) to treat aggression in ADHD patients, as shown by a statistically significant reduction in the CGI-Severity score (Figure 1A) and CGI-Improvement score (Figure 1B) from visit 1 (baseline, pre-treatment) to visit 7 (8 weeks treatment with vafidemstat), as described in more detail in Example 3. Data is represented as meant standard error of the mean (SEM); p=0.0043 (CGI-Severity) and p=0.0352 (CGI-Improvement).

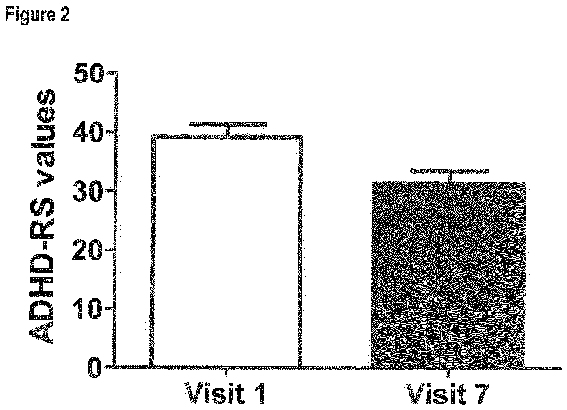

Figure 2 shows the efficacy of the KDM1A inhibitor vafidemstat to treat ADHD, as shown by a statistically significant reduction in the Attention Deficit Hyperactivity Disorder Rating Scale (ADHD-RS) score from visit 1 (baseline, pre-treatment) to visit 7 (8 weeks treatment with vafidemstat), as described in more detail in Example 3. Data is represented as meant SEM; p=0.0279.

### DETAILED DESCRIPTION OF THE INVENTION

The invention is based on the finding that KDM1A inhibitors are useful as therapeutic agents to treat ADHD, including adult ADHD. KDM1A inhibitors, including vafidemstat, have been reported to be useful to reduce aggressiveness, such as aggressiveness associated with a disease, without sedative effects. Vafidemstat is currently in a Phase IIa clinical trial treating aggression in patients with Alzheimer's disease, Lewy Body dementia, autistic spectrum disorder, ADHD and borderline personality disorder (REIMAGINE trial). Results of this clinical trial unexpectedly demonstrated that vafidemstat is not only effective to treat aggression in ADHD patients, but exhibits additional therapeutic effects on ADHD, as detailed below and in the Examples. KDM1A inhibitors and particularly vafidemstat are useful as a treatment for ADHD, including adult ADHD.

Accordingly, the present invention provides a KDM1A inhibitor for use in the treatment of ADHD as further defined in the claims.

The present invention provides a KDM1A inhibitor for use in the treatment of ADHD by treating (e.g. alleviating or improving) one or more non-aggressive symptoms of ADHD.

In accordance with the present invention, "non-aggressive" as for example used in the context of a ADHD symptom means that said symptom of ADHD is not directly related to or associated with aggression or aggressive behavior. "Aggression", "aggressive" and related terms, as used herein, refer to any kind of abnormal, pathological or inappropriate aggressive or violent behavior, hostility or agitation, for example physical or verbal, including interpersonal aggressiveness (i.e. towards other subjects) and/or intrapersonal aggressiveness (i.e. self-aggressiveness).

Examples of non-aggressive symptoms of ADHD include: not listening when directly spoken to, difficulty organizing things, losing things, forgetful in daily activities, easily distracted, fidgeting, difficulty awaiting turn, difficulty sustaining attention, difficulty with sustained mental effort, difficulty following instructions, difficulty remaining seated and talking excessively.

In some embodiments, the present invention provides a KDM1A inhibitor for use in the treatment of ADHD by treating (e.g. alleviating or improving) one or more non-aggressive symptoms of ADHD and by treating (e.g. reducing) aggression.

In some embodiments, the present invention provides a KDM1A inhibitor for use in the treatment of ADHD by treating (e.g. alleviating or improving) one or more non-aggressive symptoms of ADHD and by treating (e.g. reducing) agitation.

In some embodiments, the present invention provides a KDM1A inhibitor for use in the treatment of ADHD by treating (e.g. alleviating or improving) one or more non-aggressive symptoms of ADHD and by treating (e.g. reducing) agitation and aggression.

In some embodiments, the present invention provides a KDM1A inhibitor for use in the treatment of an ADHD patient by treating (e.g. alleviating or improving) one or more non-aggressive symptoms of ADHD.

In some embodiments, the present invention provides a KDM1A inhibitor for use in the treatment of one or more non-aggressive symptoms of ADHD.

In some embodiments, the present invention provides a KDM1A inhibitor for use in the treatment of one or more non-aggressive symptoms of ADHD as well as agitation and/or aggression.

The KDM1A inhibitor for use in the methods and uses of the invention is the compound 5-((((1R,2S)-2-(4-(benzyloxy)phenyl)cyclopropyl)amino)methyl)-1,3,4-oxadiazol-2-amine, also known as (41R,42S)-6-oxa-3-aza-1(2)-[1,3,4]oxadiazola-5(1,4),8(1)-dibenzena-4(1,2)-cyclopropanaoctaphan-15-amine, vafidemstat (INN) or ORY-2001, or a pharmaceutically acceptable salt or solvate thereof, and it is particularly preferred that the KDM1A inhibitor is the compound 5-((((1R,2S)-2-(4-(benzyloxy)phenyl)cyclopropyl)amino)methyl)-1,3,4-oxadiazol-2-amine (in non-salt form). The names "5-((((1R,2S)-2-(4-(benzyloxy)phenyl)cyclopropyl)amino)methyl)-1,3,4-oxadiazol-2-amine", "(41R,42S)-6-oxa-3-aza-1(2)-[1,3,4]oxadiazola-5(1,4),8(1)-dibenzena-4(1,2)-cyclopropanaoctaphan-15-amine", "vafidemstat" or "ORY-2001" are used herein interchangeably.

The present invention provides vafidemstat, or a pharmaceutically acceptable salt or solvate thereof, for use in the treatment of ADHD by treating (e.g. alleviating or improving) one or more non-aggressive symptoms of ADHD.

In some embodiments, the present invention provides vafidemstat, or a pharmaceutically acceptable salt or solvate thereof, for use in the treatment of ADHD by treating (e.g. alleviating or improving) one or more non-aggressive symptoms of ADHD and by treating (e.g. reducing) aggression.

In some embodiments, the present invention provides vafidemstat, or a pharmaceutically acceptable salt or solvate thereof, for use in the treatment of ADHD by treating (e.g. alleviating or improving) one or more non-aggressive symptoms of ADHD and by treating (e.g. reducing) agitation.

In some embodiments, the present invention provides vafidemstat, or a pharmaceutically acceptable salt or solvate thereof, for use in the treatment of ADHD by treating (e.g. alleviating or improving) one or more non-aggressive symptoms of ADHD and by treating (e.g. reducing) agitation and aggression.

In some embodiments, the present invention provides vafidemstat, or a pharmaceutically acceptable salt or solvate thereof, for use in the treatment of an ADHD patient by treating (e.g. alleviating or improving) one or more non-aggressive symptoms of ADHD.

In some embodiments, the present invention provides vafidemstat, or a pharmaceutically acceptable salt or solvate thereof, for use in the treatment of an ADHD patient by treating (e.g. alleviating or improving) one or more non-aggressive symptoms of ADHD and by treating (e.g. reducing) aggression.

In some embodiments, the present invention provides vafidemstat, or a pharmaceutically acceptable salt or solvate thereof, for use in the treatment of an ADHD patient by treating (e.g. alleviating or improving) one or more non-aggressive symptoms of ADHD and by treating (e.g. reducing) agitation.

In some embodiments, the present invention provides vafidemstat, or a pharmaceutically acceptable salt or solvate thereof, for use in the treatment of an ADHD patient by treating (e.g. alleviating or improving) one or more non-aggressive symptoms of ADHD and by treating (e.g. reducing) agitation and aggression.

In some embodiments, the present invention provides vafidemstat, or a pharmaceutically acceptable salt or solvate thereof, for use in the treatment of one or more non-aggressive symptoms of ADHD, as further defined in the claims.

In some embodiments, the present invention provides vafidemstat, or a pharmaceutically acceptable salt or solvate thereof, for use in the treatment of one or more non-aggressive symptoms of ADHD, as defined in the claims, as well as agitation and/or aggression.

In some embodiments, the ADHD is adult ADHD.

Preferably, the KDM1A inhibitor for use in the herein described methods of treatment and uses, for example vafidemstat (or a pharmaceutically acceptable salt or solvate thereof), is administered orally. Exemplary formulations which can be administered via peroral ingestion are described in more detail further below.

The present invention provides the compound vafidemstat, or a pharmaceutically acceptable salt or solvate thereof, for use in the treatment of ADHD, as defined in the claims. Accordingly, the invention relates to the compound vafidemstat as a free base (in non-salt form) for use in the treatment of ADHD and, furthermore, the invention also relates to a pharmaceutically acceptable salt or solvate of vafidemstat for use in the treatment of ADHD.

As illustrated in the Examples, it has been unexpectedly found in the context of the present invention that KDM1A inhibitors such as e.g. vafidemstat are useful to treat ADHD, such as adult ADHD. As part of a Phase IIa clinical trial evaluating the KDM1A inhibitor vafidemstat as a treatment for aggression in human patients with a range of CNS disorders, it has been shown that KDM1A inhibitors such as vafidemstat produce a significant improvement on aggressive behavior in ADHD patients. As illustrated in Example 3 and Figures 1A and 1B, treatment with the KDM1A inhibitor vafidemstat causes an improvement in the CGI Severity (CGI-S) (Figure 1A) and CGI-Improvement (CGI-I) (Figure 1B) scales used to measure aggression in ADHD patients after 8 weeks of treatment. The Clinical Global Impression (CGI) values reflect physician's ratings of patients' aggression severity (CGI-S) and improvement in aggression (CGI-I). As explained in greater detail in Example 3 and illustrated in Figure 2, using a validated scale specifically designed for ADHD and widely used to evaluate ADHD in adults, the ADHD-RS scale, it has been surprisingly found that treatment with a KDM1A inhibitor such as vafidemstat also produces therapeutic effects on ADHD independent to or beyond its effects on aggression, as shown by a statistically significant improvement on the overall ADHD-RS scale score comparing the ADHD-RS total score after 8 weeks of treatment with vafidemstat (score at visit 7) with the score at baseline, prior to starting treatment with vafidemstat (score at visit 1). The ADHD-RS is a scale of 18 items which reflects DSM-5 criteria for ADHD, and consists of a lack of attention sub-scale (9 items) and another subscale on hyperactivity/impulsiveness (9 items). As none of the 18 items in the ADHD-RS is related or specifically aimed to the evaluation of aggressive behavior, the results obtained in ADHD patients using the ADHD-RS scale show that KDM1A inhibitors including vafidemstat have a broad therapeutic effect in ADHD, with therapeutic effects to treat ADHD beyond treating aggresion. KDM1A inhibitors like vafidemstat can thus be used to treat ADHD, including treating core features of ADHD as defined herein.

### KDM1A inhibitors

As used herein, a KDM1A inhibitor is a compound which inhibits KDM1A, particularly human KDM1A.

Both irreversible and reversible KDM1A inhibitors have been reported. Irreversible KDM1A inhibitors exert their inhibitory activity by becoming covalently bound to the FAD cofactor within the KDM1A active site and are generally based on a 2-cyclyl-cyclopropylamino moiety such as a 2-(hetero)arylcyclopropylamino moiety. Reversible inhibitors of KDM1A have also been disclosed.

Non-limiting examples of KDM1A inhibitors are disclosed e.g. in: WO2010/043721, WO2010/084160, WO2011/035941, WO2011/042217, WO2011/131697, WO2012/013727, WO2012/013728, WO2012/045883, WO2013/057320, WO2013/057322, WO2010/143582, US2010-0324147, WO2011/022489, WO2011/131576, WO2012/034116, WO2012/135113, WO2013/022047, WO2013/025805, WO2014/058071, WO2014/084298, WO2014/086790, WO2014/164867, WO2014/205213, WO2015/021128, WO2015/031564, US2015-0065434, WO2007/021839, WO2008/127734, WO2015/089192, CN104119280, CN103961340, CN103893163, CN103319466, CN103054869, WO2015/123408, WO2015/123424, WO2015/123437, WO2015/123465, WO2015/156417, WO2015/181380, WO2016/123387, WO2016/130952, WO2016/172496, WO2016/177656, WO2017/027678, CN106045862, WO2012/071469, WO2013/033688, WO2014/085613, WO2015/120281, WO2015/134973, WO2015/168466, WO2015/200843, WO2016/003917, WO2016/004105, WO2016/007722, WO2016/007727, WO2016/007731, WO2016/007736, WO2016/034946, WO2016/037005, WO2016/161282, WO2017/004519, WO2017/027678, WO2017/079476, WO2017/079670, WO2017/090756, WO2017/109061, WO2017/116558, WO2017/114497, CN106432248, CN106478639, CN106831489, CN106928235, CN105985265, WO2017/149463, WO2017/157322, WO2017/195216, WO2017/198780, WO2017/215464, WO2018/081342, WO2018/081343, US2017-0283397, WO2019/009412, WO2018/234978, WO2018/226053, WO2018/216800, WO2018/213211, WO2018/137644, as well as 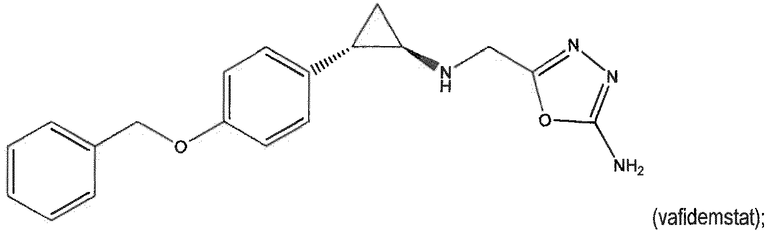 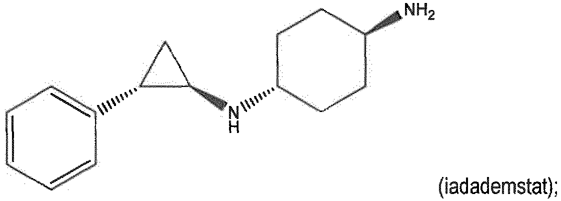 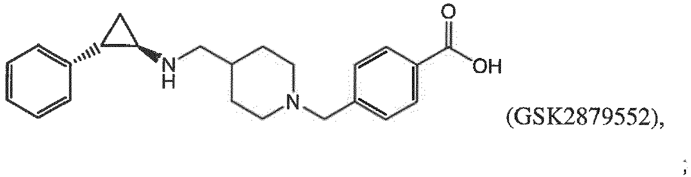 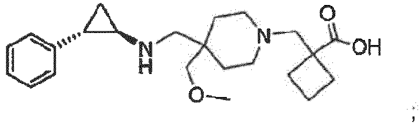 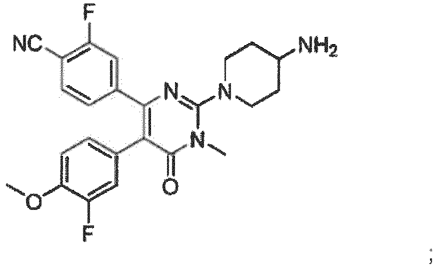 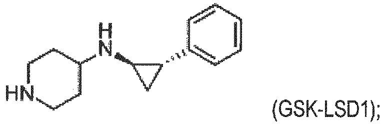 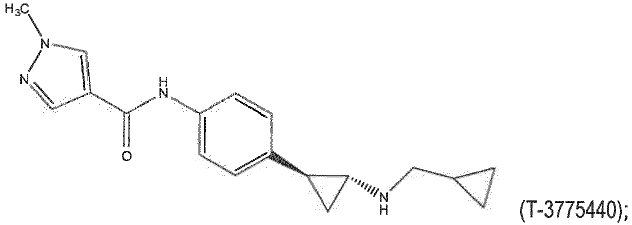 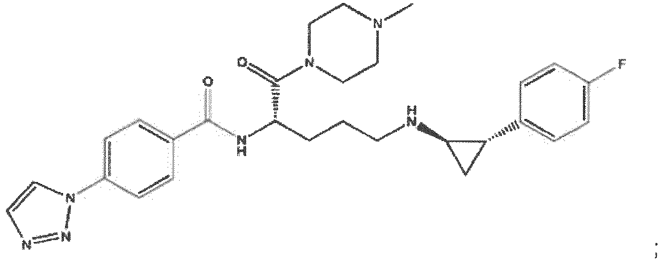 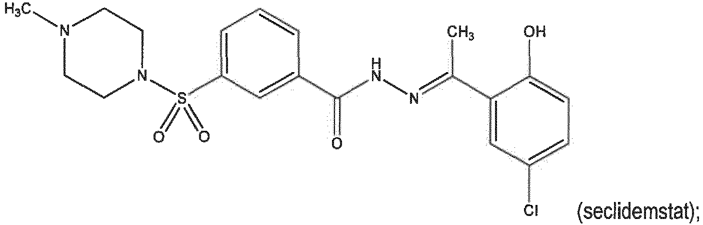 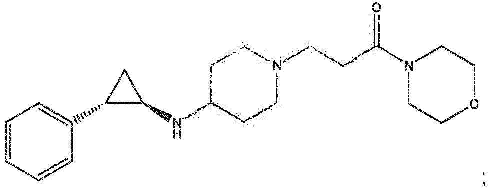 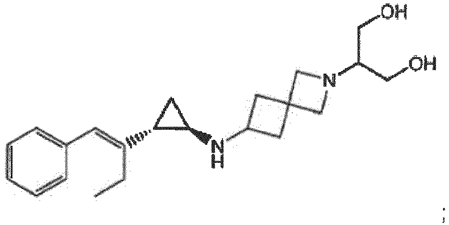 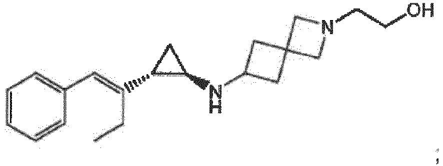 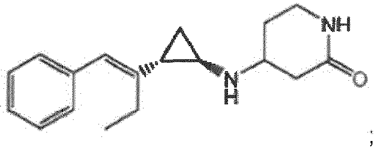
5-{(1R,2R)-2-[(Cyclopropylmethyl)amino]cyclopropyl}-N-(tetrahydro-2H-pyran-4-yl)thiophene-3-carboxamide (TAK-418);
3-((1S,2R)-2-(cyclobutylamino)cyclopropyl)-N-(5-methyl-1,3,4-thiadiazol-2-yl)benzamide (T-448); or 3-((1S,2R)-2-(cyclopropylamino)cyclopropyl)-N-(5-methyl-1,3,4-thiadiazol-2-yl)benzamide;
including any optically active stereoisomer thereof, or any pharmaceutically acceptable salt or solvate thereof. Any one of the above-depicted compounds comprising a 1,2-substituted cyclopropyl ring can be employed in the form of the corresponding trans-isomer (wherein the two substituents at the cyclopropyl ring are in trans-configuration), or in the form of any one of the respective specific trans-isomers (wherein the two substituents at the cyclopropyl ring have the same absolute configuration as shown in the drawn structure; or wherein the two substituents at the cyclopropyl ring each have the opposite absolute configuration as shown in the drawn structure).

Further non-limiting examples of KDM1A inhibitors are disclosed e.g. in: K Taeko et al, Bioorg Med Chem Lett 2015, 25(9):1925-8. doi: 10.1016/j.bmcl.2015.03.030. Epub 2015 Mar 20, PMID: 25827526; S Valente et al, Eur J Med Chem. 2015, 94:163-74. doi: 10.1016/j.ejmech.2015.02.060. Epub 2015 Mar 3, PMID:25768700; MN Ahmed Khan et al Med. Chem. Commun., 2015,6, 407-412, DOI: 10.1039/C4MD00330F epub 29 Sep 2014; M Pieroni et al, Eur J Med Chem. 2015 ;92:377-386. doi: 10.1016/j.ejmech.2014.12.032. Epub 2015 Jan 7. PMID:25585008; V Rodriguez et al, Med. Chem. Commun., 2015,6, 665-670 DOI: 10.1039/C4MD00507D, Epub 23 Dec 2014; P Vianello et al, Eur J Med Chem. 2014, 86:352-63. doi: 10.1016/j.ejmech.2014.08.068. Epub 2014 Aug 27; DP Mould et al, Med. Res. Rev., 2015,35:586-618. doi:10.1002/med.21334, epub 24-nov-2014; LY Ma et al, 2015, 58(4):1705-16. doi: 10.1021/acs.jmedchem.5b00037. Epub 2015 Feb 6; SL Nowotarski et al, 2015, 23(7):1601-12. doi: 10.1016/j.bmc.2015.01.049. Epub 2015 Feb 7. PMID:25725609; CJ Kutz et al Medchemcomm. 2014, 5(12):1863-1870 PMID: 25580204; C Zhou et al, Chemical Biology & Drug Design,2015, 85(6):659-671. doi:10.1111/cbdd.12461, epub 22-dec-2014; P Prusevich et al, ACS Chem Biol. 2014, 9(6):1284-93. doi: 10.1021/cb500018s. Epub 2014 Apr 7; B Dulla et al, Org Biomol Chem 2013,11, 3103-3107, doi: 10.1039/c3ob40217g; JR Hitchin et al, MedChemCommun,2013, 4, 1513-1522 DOI: 10.1039/c3md00226h; and Y Zhou et al, Biorg Med Chem Lett, 2015, online publication 20-Jun-2015, doi:10.1016/j.bmcl.2015.06.054. Irreversible KDM1A inhibitors include, without limitation, any one of the compounds disclosed in: WO2010/043721, WO2010/084160, WO2011/035941, WO2011/042217, WO2011/131697, WO2012/013727, WO2012/013728, WO2012/045883, WO2013/057320, WO2013/057322, WO2010/143582, US2010-0324147, WO2011/131576, WO2012/135113, WO2013/022047, WO2014/058071, WO2014/084298, WO2014/086790, WO2014/164867, WO2015/021128; WO2015/123408, WO2015/123424, WO2015/123437, WO2015/123465, WO2015/156417, WO2015/181380, WO2016/123387, WO2016/130952, WO2016/172496, WO2016/177656, WO2017/027678, CN106045862, WO2014/164867 WO2017/027678, WO2017/079476, WO2017/109061, WO2017/116558, WO2017/114497, CN106831489; WO2018/137644, WO2018/226053, WO2019/009412, K Taeko et al, Bioorg Med Chem Lett. 2015, 25(9):1925-8. doi: 10.1016/j.bmcl.2015.03.030. Epub 2015 Mar 20, PMID: 25827526; S Valente et al, Eur J Med Chem. 2015, 94:163-74. doi: 10.1016/j.ejmech.2015.02.060. Epub 2015 Mar 3, PMID:25768700; MN Ahmed Khan et al Med. Chem. Commun., 2015,6, 407-412, DOI: 10.1039/C4MD00330F epub 29 Sep 2014; M Pieroni et al, Eur J Med Chem. 2015 ;92:377-386. doi: 10.1016/j.ejmech.2014.12.032. Epub 2015 Jan 7. PMID:25585008; V Rodriguez et al, Med. Chem. Commun., 2015,6, 665-670 DOI: 10.1039/C4MD00507D, Epub 23 Dec 2014; or P Vianello et al, Eur J Med Chem. 2014, 86:352-63. doi: 10.1016/j.ejmech.2014.08.068. Epub 2014 Aug 27, as well as 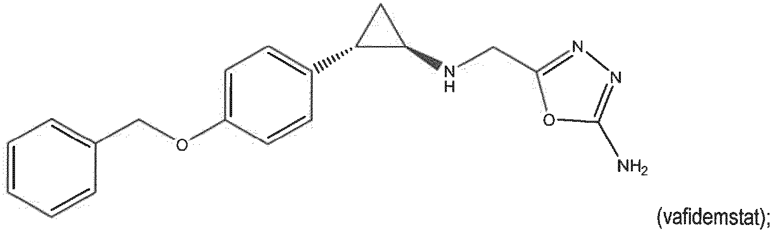 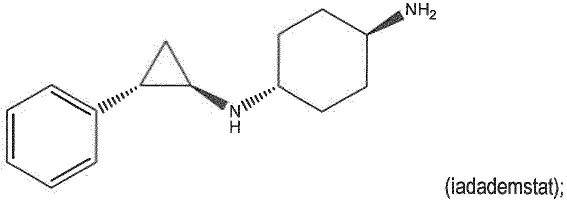 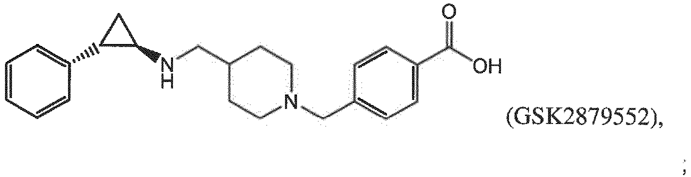 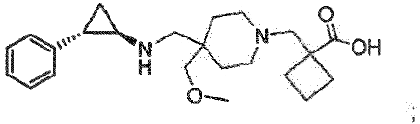 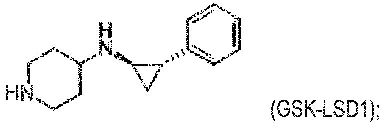 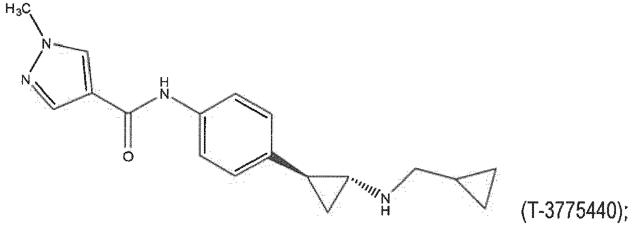 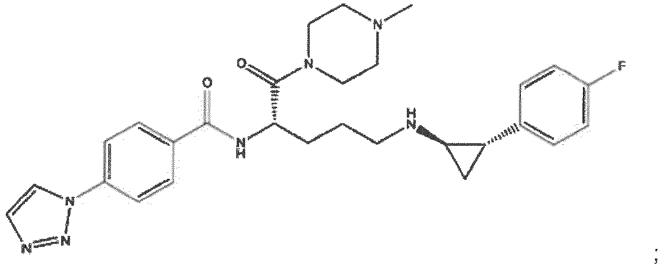 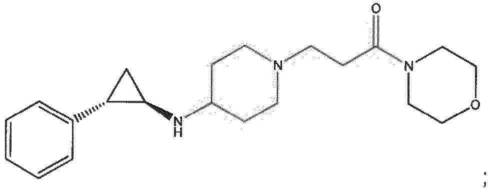 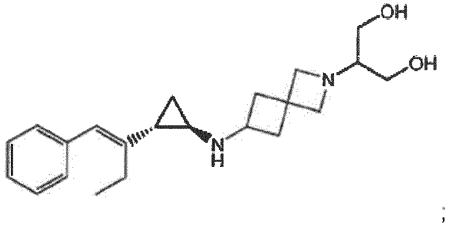 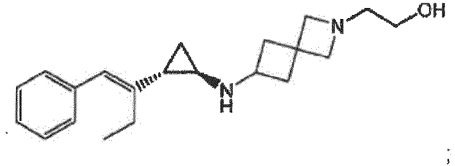 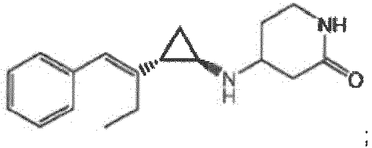
5-{(1R,2R)-2-[(Cyclopropylmethyl)amino]cyclopropyl}-N-(tetrahydro-2H-pyran-4-yl)thiophene-3-carboxamide (TAK-418);
3-((1S,2R)-2-(cyclobutylamino)cyclopropyl)-N-(5-methyl-1,3,4-thiadiazol-2-yl)benzamide (T-448); or 3-((1S,2R)-2-(cyclopropylamino)cyclopropyl)-N-(5-methyl-1,3,4-thiadiazol-2-yl)benzamide;
including any optically active stereoisomer thereof, or any pharmaceutically acceptable salt or solvate thereof. Any one of the above-depicted compounds comprising a 1,2-substituted cyclopropyl ring can be employed in the form of the corresponding trans-isomer (wherein the two substituents at the cyclopropyl ring are in trans-configuration), or in the form of any one of the respective specific trans-isomers (wherein the two substituents at the cyclopropyl ring have the same absolute configuration as shown in the drawn structure; or wherein the two substituents at the cyclopropyl ring each have the opposite absolute configuration as shown in the drawn structure).

Reversible KDM1A inhibitors include, without limitation, any one of the compounds disclosed in WO2007/021839, WO2008/127734, WO2011/022489, WO2012/034116, WO2012/071469, WO2013/025805, US2015/0065434, WO2013/033688, CN103054869, CN103319466, WO2014/085613, CN103893163A, CN103961340, WO2014/205213, WO2015/031564, WO2015/089192, WO2015/120281, WO2015/134973, WO2015/168466, WO2015/200843, WO2016/003917, WO2016/004105, WO2016/007722, WO2016/007727, WO2016/007731, WO2016/007736, WO2016/034946, WO2016/037005, WO2016/161282, WO2017/004519, WO2017/079670, WO2017/080756, CN106432248, CN106478639, CN106928235, WO2018/234978, WO2018/216800, WO2018/213211, as well as 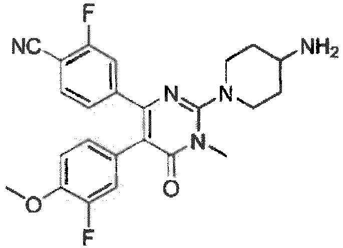 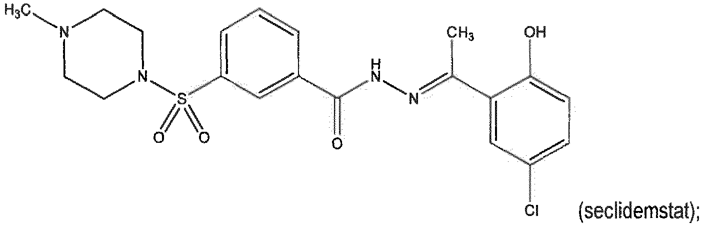 including any optically active stereoisomer thereof, or any pharmaceutically acceptable salt or solvate thereof.

The ability of a compound to inhibit KDM1A can be tested in vitro using any method to determine KDM1A inhibition known in the art, for example the method disclosed in Example 2.

The KDM1A inhibitor for use in the methods and uses according to the invention is vafidemstat (i.e. 5-((((1R,2S)-2-(4-(benzyloxy)phenyl)cyclopropyl)amino)methyl)-1,3,4-oxadiazol-2-amine), or a pharmaceutically acceptable salt or solvate thereof.

Other KDM1A inhibitors include: 5-{(1R,2R)-2-[(Cyclopropylmethyl)amino]cyclopropyl}-N-(tetrahydro-2H-pyran-4-yl)thiophene-3-carboxamide (TAK-418);
3-((1S,2R)-2-(cyclobutylamino)cyclopropyl)-N-(5-methyl-1,3,4-thiadiazol-2-yl)benzamide (T-448);
3-((1S,2R)-2-(cyclopropylamino)cyclopropyl)-N-(5-methyl-1,3,4-thiadiazol-2-yl)benzamide;
(trans)-N1-((1R,2S)-2-phenylcyclopropyl)cyclohexane-1,4-diamine (iadademstat);
(cis)-N1-((1S,2R)-2-phenylcyclopropyl)cyclohexane-1,4-diamine;
(trans)-N1-((1S,2R)-2-phenylcyclopropyl)cyclohexane-1,4-diamine;
(cis)-N1-((1R,2S)-2-phenylcyclopropyl)cyclohexane-1,4-diamine;
N1-((trans)-2-(thiazol-5-yl)cyclopropyl)cyclohexane-1,4-diamine;
N1-((trans)-2-(pyridin-3-yl)cyclopropyl)cyclohexane-1,4-diamine;
N1-((trans)-2-(6-(3-(trifluoromethyl)phenyl)pyridin-3-yl)cyclopropyl)cyclohexane-1,4-diamine;
N1-((trans)-2-(3'-(trifluoromethyl)-[1,1'-biphenyl]-4-yl)cyclopropyl)cyclohexane-1,4-diamine;
N1-((trans)-2-(4-(benzyloxy)phenyl)cyclopropyl)cyclohexane-1,4-diamine;
4-(((trans)-2-(6-(3-(trifluoromethyl)phenyl)pyridin-3-yl)cyclopropyl)amino)cyclohexanol;
4-(((trans)-2-(6-(3-(trifluoromethyl)phenyl)pyridin-3-yl)cyclopropyl)amino)cyclohexanecarboxamide;
N-(4-(((trans)-2-(6-(3-(trifluoromethyl)phenyl)pyridin-3-yl)cyclopropyl)amino)cyclohexyl)acetamide;
N-(4-(((trans)-2-(6-(3-(trifluoromethyl)phenyl)pyridin-3-yl)cyclopropyl)amino)cyclohexyl)methanesulfonamide;
(R)-1-(4-(((trans)-2-phenylcyclopropyl)amino)cyclohexyl)pyrrolidin-3-amine;
N1-((trans)-2-(4'-chloro-[1,1'-biphenyl]-4-yl)cyclopropyl)cyclohexane-1,4-diamine;
N1-((trans)-2-(3'-chloro-[1,1'-biphenyl]-4-yl)cyclopropyl)cyclohexane-1,4-diamine;
4'-((trans)-2-((4-aminocyclohexyl)amino)cyclopropyl)-[1,1'-biphenyl]-3-ol;
N-(4'-((trans)-2-((4-aminocyclohexyl)amino)cyclopropyl)-[1,1'-biphenyl]-3-yl)methanesulfonamide;
N1-((trans)-2-(4-((2-fluorobenzyl)oxy)phenyl)cyclopropyl)cyclohexane-1,4-diamine;
N1-((trans)-2-(4-((3-fluorobenzyl)oxy)phenyl)cyclopropyl)cyclohexane-1,4-diamine;
N1-((trans)-2-(4-((4-fluorobenzyl)oxy)phenyl)cyclopropyl)cyclohexane-1,4-diamine;
N1-methyl-N4-((trans)-2-phenylcyclopropyl)cyclohexane-1,4-diamine;
N1-methyl-N4-((trans)-2-(3'-(trifluoromethyl)-[1,1'-biphenyl]-4-yl)cyclopropyl)cyclohexane-1,4-diamine;
N1-((trans)-2-(4-(benzyloxy)phenyl)cyclopropyl)-N4-methylcyclohexane-1,4-diamine;
N1-((trans)-2-phenylcyclopropyl)cyclobutane-1,3-diamine;
N 1-((trans)-2-(3'-(trifluoromethyl)-[1,1'-biphenyl]-4-yl)cyclopropyl)cyclobutane-1,3-diamine;
N1-((trans)-2-(4-(benzyloxy)phenyl)cyclopropyl)cyclobutane-1,3-diamine;
N1-((trans)-2-phenylcyclopropyl)-2,3-dihydro-1H-indene-1,3-diamine;
N1-((trans)-2-(3'-(trifluoromethyl)-[1,1'-biphenyl]-4-yl)cyclopropyl)-2,3-dihydro-1H-indene-1,3-diamine;
N1-((trans)-2-(4-(benzyloxy)phenyl)cyclopropyl)-2,3-dihydro-1H-indene-1,3-diamine;
N1-((trans)-2-fluoro-2-phenylcyclopropyl)cyclohexane-1,4-diamine;
N1-((1S,2S)-2-fluoro-2-phenylcyclopropyl)cyclohexane-1,4-diamine;
N1-((1R,2R)-2-fluoro-2-phenylcyclopropyl)cyclohexane-1,4-diamine;
1-methyl-N4-((trans)-2-phenylcyclopropyl)cyclohexane-1,4-diamine;
4-(aminomethyl)-N-((trans)-2-phenylcyclopropyl)cyclohexanamine;
N1-((trans)-2-phenylcyclopropyl)cyclohexane-1,3-diamine;
N1-((cis)-2-phenylcyclopropyl)cyclohexane-1,4-diamine;
Tert-butyl (4-(((trans)-2-phenylcyclopropyl)amino)cyclohexyl)carbamate;
1-ethyl-3-(4-(((trans)-2-phenylcyclopropyl)amino)cyclohexyl)urea;
4-morpholino-N-((trans)-2-phenylcyclopropyl)cyclohexanamine;
N1-((trans)-2-(4-bromophenyl)cyclopropyl)cyclohexane-1,4-diamine;
N1-(2-(o-tolyl)cyclopropyl)cyclohexane-1,4-diamine;
N1-(2-(4-(trifluoromethyl)phenyl)cyclopropyl)cyclohexane-1,4-diamine;
N1-(2-(4-methoxyphenyl)cyclopropyl)cyclohexane-1,4-diamine;
4-(2-((4-aminocyclohexyl)amino)cyclopropyl)phenol;
N1-(2-(2-fluorophenyl)cyclopropyl)cyclohexane-1,4-diamine;
N1-(2-(3,4-difluorophenyl)cyclopropyl)cyclohexane-1,4-diamine;
N1-(2-(naphthalen-2-yl)cyclopropyl)cyclohexane-1,4-diamine;
N1-(2-methyl-2-phenylcyclopropyl)cyclohexane-1,4-diamine;
(R)-1-(4-(((trans)-2-(3'-(trifluoromethyl)-[1,1'-biphenyl]-4-yl)cyclopropyl) amino)cyclohexyl)pyrrolidin-3-amine;
(Cis)-N1-((1S,2R)-2-(3'-(trifluoromethyl)-[1,1'-biphenyl]-4-yl)cyclopropyl)cyclohexane-1,4-diamine;
(Trans)-N1-((1S,2R)-2-(3'-(trifluoromethyl)-[1,1'-biphenyl]-4-yl)cyclo-propyl)cyclohexane-1,4-diamine;
(Cis)-N1-((1R,2S)-2-(3'-(trifluoromethyl)-[1,1'-biphenyl]-4-yl)cyclo-propyl)cyclohexane-1,4-diamine;
(Trans)-N1-((1R,2S)-2-(3'-(trifluoromethyl)-[1,1'-biphenyl]-4-yl)cyclo-propyl)cyclohexane-1,4-diamine;
N1-((trans)-2-(4-cyclopropylphenyl)cyclopropyl)cyclohexane-1,4-diamine;
N1-((trans)-2-(4-(pyridin-3-yl)phenyl)cyclopropyl)cyclohexane-1,4-diamine;
N1-((trans)-2-(4-(1H-indazol-6-yl)phenyl)cyclopropyl)cyclohexane-1,4-diamine;
N1-((trans)-2-(4-(1H-pyrazol-5-yl)phenyl)cyclopropyl)cyclohexane-1,4-diamine;
3-(5-((trans)-2-((4-aminocyclohexyl)amino)cyclopropyl)thiophen-2-yl)phenol;
3-(5-((trans)-2-((4-aminocyclohexyl)amino)cyclopropyl)thiazol-2-yl)phenol;
3-(5-((trans)-2-((4-aminocyclohexyl)amino)cyclopropyl)pyridin-2-yl)-5-methoxybenzonitrile;
5-(5-((trans)-2-((4-aminocyclohexyl)amino)cyclopropyl)pyridin-2-yl)-2-methylphenol;
N-(4'-((trans)-2-((4-aminocyclohexyl)amino)cyclopropyl)-6-methoxy-[1,1'-biphenyl]-3-yl)methanesulfonamide;
N-(3-(5-((trans)-2-((4-aminocyclohexyl)amino)cyclopropyl)thiazol-2-yl)phenyl)-2-cyanobenzenesulfonamide ;
N-(4'-((trans)-2-((4-aminocyclohexyl)amino)cyclopropyl)-[1,1'-biphenyl]-3-yl)-2-cyanobenzenesulfonamide;
6-amino-N-(4'-((trans)-2-((4-aminocyclohexyl)amino)cyclopropyl)-[1,1'-biphenyl]-3-yl)pyridine-3-sulfonamide;
N-(4'-((trans)-2-((4-aminocyclohexyl)amino)cyclopropyl)-[1,1'-biphenyl]-3-yl)piperazine-1-sulfonamide;
N1-((cis)-2-fluoro-2-phenylcyclopropyl)cyclohexane-1,4-diamine;
N1-((trans)-2-(4-((3-(piperazin-1-yl)benzyl)oxy)phenyl)cyclopropyl)cyclohexane-1,4-diamine;
N1-((trans)-2-(4-(pyridin-3-ylmethoxy)phenyl)cyclopropyl)cyclohexane-1,4-diamine;
N1-((trans)-2-(6-((3-methylbenzyl)amino)pyridin-3-yl)cyclopropyl)cyclohexane-1,4-diamine;
3-((5-((trans)-2-((4-aminocyclohexyl)amino)cyclopropyl)pyridin-2-yl) amino)benzonitrile;
N1-((trans)-2-(naphthalen-2-yl)cyclopropyl)cyclohexane-1,4-diamine;
N1-((trans)-2-(o-tolyl)cyclopropyl)cyclohexane-1,4-diamine;
N1-((trans)-2-(4-(trifluoromethyl)phenyl)cyclopropyl)cyclohexane-1,4-diamine;
N1-((trans)-2-(4-methoxyphenyl)cyclopropyl)cyclohexane-1,4-diamine;
N1-((trans)-2-(2-fluorophenyl)cyclopropyl)cyclohexane-1,4-diamine;
N1-((trans)-2-(3,4-difluorophenyl)cyclopropyl)cyclohexane-1,4-diamine;
N1-((trans)-2-methyl-2-phenylcyclopropyl)cyclohexane-1,4-diamine;
(cis)-N1-((1S,2R)-2-(pyridin-3-yl)cyclopropyl)cyclohexane-1,4-diamine ;
(trans)-N1-((1R,2S)-2-(pyridin-3-yl)cyclopropyl)cyclohexane-1,4-diamine;
(cis)-N1-((1R,2S)-2-(pyridin-3-yl)cyclopropyl)cyclohexane-1,4-diamine;
(trans)-N1-((1S,2R)-2-(pyridin-3-yl)cyclopropyl)cyclohexane-1,4-diamine;
(cis)-N1-((1S,2R)-2-phenylcyclopropyl)cyclobutane-1,3-diamine ;
(trans)-N1-((1R,2S)-2-phenylcyclopropyl)cyclobutane-1,3-diamine;
(cis)-N1-((1R,2S)-2-phenylcyclopropyl)cyclobutane-1,3-diamine ;
(trans)-N1-((1S,2R)-2-phenylcyclopropyl)cyclobutane-1,3-diamine;
(cis)-N1-((1S,2R)-2-(3,4-difluorophenyl)cyclopropyl)cyclohexane-1,4-diamine;
(trans)-N1-((1R,2S)-2-(3,4-difluorophenyl)cyclopropyl)cyclohexane-1,4-diamine;
(cis)-N1-((1R,2S)-2-(3,4-difluorophenyl)cyclopropyl)cyclohexane-1,4-diamine;
(trans)-N1-((1S,2R)-2-(3,4-difluorophenyl)cyclopropyl)cyclohexane-1,4-diamine;
(cis)-N1-((1S,2R)-2-(naphthalen-2-yl)cyclopropyl)cyclohexane-1,4-diamine;
(trans)-N1-((1R,2S)-2-(naphthalen-2-yl)cyclopropyl)cyclohexane-1,4-diamine;
(cis)-N1-((1R,2S)-2-(naphthalen-2-yl)cyclopropyl)cyclohexane-1,4-diamine;
(trans)-N1-((1S,2R)-2-(naphthalen-2-yl)cyclopropyl)cyclohexane-1,4-diamine;
(cis)-N1-((1S,2R)-2-(4-(1H-pyrazol-5-yl)phenyl)cyclopropyl)cyclohexane-1,4-diamine;
(trans)-N1-((1R,2S)-2-(4-(1H-pyrazol-5-yl)phenyl)cyclopropyl)cyclohexane-1,4-diamine;
(cis)-N1-((1R,2S)-2-(4-(1H-pyrazol-5-yl)phenyl)cyclopropyl)cyclohexane-1,4-diamine;
(trans)-N1-((1S,2R)-2-(4-(1H-pyrazol-5-yl)phenyl)cyclopropyl)cyclohexane-1,4-diamine;
N-(4'-((1R,2S)-2-(((cis)-4-aminocyclohexyl)amino)cyclopropyl)-[1,1'-biphenyl]-3-yl)piperazine-1-sulfonamide;
N-(4'-((1S,2R)-2-(((trans)-4-aminocyclohexyl)amino)cyclopropyl)-[1,1'-biphenyl]-3-yl)piperazine-1-sulfonamide;
N-(4'-((1S,2R)-2-(((cis)-4-aminocyclohexyl)amino)cyclopropyl)-[1,1'-biphenyl]-3-yl)piperazine-1-sulfonamide;
N-(4'-((1R,2S)-2-(((trans)-4-aminocyclohexyl)amino)cyclopropyl)-[1,1'-biphenyl]-3-yl)piperazine-1-sulfonamide;
(cis)-N1-((1S,2R)-2-(4-((2-fluorobenzyl)oxy)phenyl)cyclopropyl)cyclohexane-1,4-diamine;
(trans)-N1-((1R,2S)-2-(4-((2-fluorobenzyl)oxy)phenyl)cyclopropyl)cyclohexane-1,4-diamine;
(cis)-N1-((1R,2S)-2-(4-((2-fluorobenzyl)oxy)phenyl)cyclopropyl)cyclohexane-1,4-diamine;
(trans)-N1-((1S,2R)-2-(4-((2-fluorobenzyl)oxy)phenyl)cyclopropyl)cyclohexane-1,4-diamine;
N-((trans)-2-phenylcyclopropyl)piperidin-4-amine;
N-((1S,2R)-2-phenylcyclopropyl)piperidin-4-amine;
N-((1R,2S)-2-phenylcyclopropyl)piperidin-4-amine;
N-((trans)-2-(4-(benzyloxy)phenyl)cyclopropyl)piperidin-4-amine;
N-((trans)-2-(6-(3-(trifluoromethyl)phenyl)pyridin-3-yl)cyclopropyl)tetrahydro-2H-pyran-4-amine;
N-((trans)-2-(pyridin-3-yl)cyclopropyl)piperidin-4-amine;
N-((trans)-2-(thiazol-5-yl)cyclopropyl)piperidin-4-amine;
N-((trans)-2-(3'-(trifluoromethyl)-[1,1'-biphenyl]-4-yl)cyclopropyl)piperidin-4-amine;
N-((trans)-2-phenylcyclopropyl)piperidin-3-amine;
N-((trans)-2-(3'-(trifluoromethyl)-[1,1'-biphenyl]-4-yl)cyclopropyl)piperidin-3-amine;
N-((trans)-2-(4-(benzyloxy)phenyl)cyclopropyl)piperidin-3-amine;
N-((trans)-2-phenylcyclopropyl)pyrrolidin-3-amine;
N-((trans)-2-(3'-(trifluoromethyl)-[1,1'-biphenyl]-4-yl)cyclopropyl)pyrrolidin-3-amine;
N-((trans)-2-(4-(benzyloxy)phenyl)cyclopropyl)pyrrolidin-3-amine;
N-((trans)-2-phenylcyclopropyl)azetidin-3-amine;
N-((trans)-2-(3'-(trifluoromethyl)-[1,1'-biphenyl]-4-yl)cyclopropyl)azetidin-3-amine;
N-((trans)-2-(4-(benzyloxy)phenyl)cyclopropyl)azetidin-3-amine;
N-((trans)-2-phenylcyclopropyl)azepan-3-amine;
N-((trans)-2-phenylcyclopropyl)-8-azabicyclo[3.2.1]octan-3-amine;
N-((trans)-2-phenylcyclopropyl)-3-azabicyclo[3.2.1]octan-8-amine;
N-((trans)-2-phenylcyclopropyl)decahydroquinolin-4-amine;
N-((trans)-2-phenylcyclopropyl)-1,2,3,4-tetrahydroquinolin-4-amine;
N-((trans)-2-phenylcyclopropyl)-3-azaspiro[5.5]undecan-9-amine;
N-((trans)-2-phenylcyclopropyl)-2-azaspiro[4.5]decan-8-amine;
N-((trans)-2-phenylcyclopropyl)-2,3-dihydrospiro[indene-1,4'-piperidin]-3-amine;
N-((1S,2R)-2-(4-(benzyloxy)phenyl)cyclopropyl)piperidin-4-amine;
N-((1R,2S)-2-(4-(benzyloxy)phenyl)cyclopropyl)piperidin-4-amine ;
N-((1S,2R)-2-(pyridin-3-yl)cyclopropyl)piperidin-4-amine;
N-((1R,2S)-2-(pyridin-3-yl)cyclopropyl)piperidin-4-amine;
N-((1S,2S)-2-(thiazol-5-yl)cyclopropyl)piperidin-4-amine;
N-((1R,2R)-2-(thiazol-5-yl)cyclopropyl)piperidin-4-amine;
N-((1S,2R)-2-(3'-(trifluoromethyl)-[1,1'-biphenyl]-4-yl)cyclopropyl)piperidin-4-amine;
N-((1R,2S)-2-(3'-(trifluoromethyl)-[1,1'-biphenyl]-4-yl)cyclopropyl)piperidin-4-amine;
N-((trans)-2-phenylcyclopropyl)-7-azaspiro[3.5]nonan-2-amine;
N-(2-(o-tolyl)cyclopropyl)piperidin-4-amine;
N-(2-(2-fluorophenyl)cyclopropyl)piperidin-4-amine;
N-(2-(3,4-difluorophenyl)cyclopropyl)piperidin-4-amine;
N-(2-(4-methoxyphenyl)cyclopropyl)piperidin-4-amine;
N-(2-(naphthalen-2-yl)cyclopropyl)piperidin-4-amine;
N-(2-methyl-2-phenylcyclopropyl)piperidin-4-amine;
N-(6-methoxy-4'-((trans)-2-(piperidin-4-ylamino)cyclopropyl)-[1,1'-biphenyl]-3-yl)methanesulfonamide;
N-(4'-((trans)-2-(piperidin-4-ylamino)cyclopropyl)-[1,1'-biphenyl]-3-yl)propane-2-sulfonamide;
1-(methylsulfonyl)-N-((trans)-2-phenylcyclopropyl)piperidin-4-amine;
1-(4-(((trans)-2-(4-bromophenyl)cyclopropyl)amino)piperidin-1-yl)ethanone;
4-(((trans)-2-(4-bromophenyl)cyclopropyl)amino)piperidine-1-carboxamide;
N-((trans)-2-(4-bromophenyl)cyclopropyl)tetrahydro-2H-pyran-4-amine;
2,2,6,6-tetramethyl-N-((trans)-2-phenylcyclopropyl)piperidin-4-amine;
1-methyl-N-((trans)-2-phenylcyclopropyl)piperidin-4-amine;
1-isopropyl-N-((trans)-2-phenylcyclopropyl)piperidin-4-amine;
N-((trans)-2-phenylcyclopropyl)-1-(2,2,2-trifluoroethyl)piperidin-4-amine;
N-((trans)-2-phenylcyclopropyl)-1-(pyridin-4-yl)piperidin-4-amine;
4-(((trans)-2-(4-bromophenyl)cyclopropyl)amino)tetrahydro-2H-thiopyran 1,1-dioxide;
N-((trans)-2-fluoro-2-phenylcyclopropyl)piperidin-4-amine;
N-((1S,2S)-2-fluoro-2-phenylcyclopropyl)piperidin-4-amine;
N-((1R,2R)-2-fluoro-2-phenylcyclopropyl)piperidin-4-amine;
N-((trans)-2-(naphthalen-2-yl)cyclopropyl)piperidin-4-amine;
N-((trans)-2-methyl-2-phenylcyclopropyl)piperidin-4-amine;
N-((trans)-2-(o-tolyl)cyclopropyl)piperidin-4-amine;
N-((trans)-2-(2-fluorophenyl)cyclopropyl)piperidin-4-amine;
N-((trans)-2-(3,4-difluorophenyl)cyclopropyl)piperidin-4-amine;
N-((trans)-2-(4-methoxyphenyl)cyclopropyl)piperidin-4-amine;
(Trans)-2-phenyl-N-(piperidin-4-ylmethyl)cyclopropanamine;
(Trans)-2-phenyl-N-(2-(piperidin-4-yl)ethyl)cyclopropanamine;
(Trans)-2-phenyl-N-(2-(tetrahydro-2H-pyran-4-yl)ethyl)cyclopropanamine;
(Trans)-2-(4'-chloro-[1,1'-biphenyl]-4-yl)-N-(2-(tetrahydro-2H-pyran-4-yl)ethyl)cyclopropanamine;
(Trans)-N-(piperidin-4-ylmethyl)-2-(pyridin-3-yl)cyclopropanamine;
(Trans)-N-(piperidin-4-ylmethyl)-2-(thiazol-5-yl)cyclopropanamine;
(Trans)-N-(piperidin-4-ylmethyl)-2-(3'-(trifluoromethyl)-[1,1'-biphenyl]-4-yl)cyclopropanamine;
(Trans)-2-(4-(benzyloxy)phenyl)-N-(piperidin-4-ylmethyl)cyclopropanamine;
(Trans)-N-(2-(piperidin-4-yl)ethyl)-2-(pyridin-3-yl)cyclopropanamine;
(Trans)-N-(2-(piperidin-4-yl)ethyl)-2-(thiazol-5-yl)cyclopropanamine;
(Trans)-N-(2-(piperidin-4-yl)ethyl)-2-(3'-(trifluoromethyl)-[1,1'-biphenyl]-4-yl)cyclopropanamine;
(Trans)-2-(4-(benzyloxy)phenyl)-N-(2-(piperidin-4-yl)ethyl)cyclopropanamine;
(1S,2R)-2-phenyl-N-(piperidin-4-ylmethyl)cyclopropanamine ;
(1R,2S)-2-phenyl-N-(piperidin-4-ylmethyl)cyclopropanamine ;
(1S,2R)-2-phenyl-N-(2-(piperidin-4-yl)ethyl)cyclopropanamine;
(1R,2S)-2-phenyl-N-(2-(piperidin-4-yl)ethyl)cyclopropanamine ;
(1S,2R)-N-(piperidin-4-ylmethyl)-2-(pyridin-3-yl)cyclopropanamine;
(1R,2S)-N-(piperidin-4-ylmethyl)-2-(pyridin-3-yl)cyclopropanamine;
(1S,2S)-N-(piperidin-4-ylmethyl)-2-(thiazol-5-yl)cyclopropanamine;
(1R,2R)-N-(piperidin-4-ylmethyl)-2-(thiazol-5-yl)cyclopropanamine;
(1S,2R)-N-(piperidin-4-ylmethyl)-2-(3'-(trifluoromethyl)-[1,1'-biphenyl]-4-yl)cyclopropanamine;
(1R,2S)-N-(piperidin-4-ylmethyl)-2-(3'-(trifluoromethyl)-[1,1'-biphenyl]-4-yl)cyclopropanamine;
(1S,2R)-2-(4-(benzyloxy)phenyl)-N-(piperidin-4-ylmethyl)cyclopropanamine;
(1R,2S)-2-(4-(benzyloxy)phenyl)-N-(piperidin-4-ylmethyl)cyclopropanamine;
(1S,2R)-N-(2-(piperidin-4-yl)ethyl)-2-(pyridin-3-yl)cyclopropanamine;
(1R,2S)-N-(2-(piperidin-4-yl)ethyl)-2-(pyridin-3-yl)cyclopropanamine;
(1S,2S)-N-(2- (piperidin-4-yl)ethyl)-2-(thiazol-5-yl)cyclopropanamine;
(1R,2R)-N-(2-(piperidin-4-yl)ethyl)-2-(thiazol-5-yl)cyclopropanamine;
(1S,2R)-N-(2-(piperidin-4-yl)ethyl)-2-(3'-(trifluoromethyl)-[1,1'-biphenyl]-4-yl)cyclopropanamine;
(1R,2S)-N-(2-(piperidin-4-yl)ethyl)-2-(3'-(trifluoromethyl)-[1,1'-biphenyl]-4-yl)cyclopropanamine;
(1S,2R)-2-(4-(benzyloxy)phenyl)-N-(2-(piperidin-4-yl)ethyl)cyclopropanamine;
(1R,2S)-2-(4-(benzyloxy)phenyl)-N-(2-(piperidin-4-yl)ethyl)cyclopropanamine;
(Trans)-2-phenyl-N-(pyrrolidin-3-ylmethyl)cyclopropanamine;
(Trans)-2-(4-((2-fluorobenzyl)oxy)phenyl)-N-(piperidin-4-ylmethyl)cyclopropanamine;
(Trans)-N-(azetidin-3-ylmethyl)-2-phenylcyclopropanamine;
(Trans)-2-(4-cyclopropylphenyl)-N-(piperidin-4-ylmethyl)cyclopropanamine;
(Trans)-N-(piperidin-4-ylmethyl)-2-(4-(pyridin-3-yl)phenyl)cyclopropanamine;
(Trans)-2-(4-(1H-pyrazol-5-yl)phenyl)-N-(piperidin-4-ylmethyl)cyclopropanamine;
(Trans)-2-(naphthalen-2-yl)-N-(piperidin-4-ylmethyl)cyclopropanamine;
2-methyl-2-phenyl-N-(piperidin-4-ylmethyl)cyclopropanamine;
(trans)-2-methyl-2-phenyl-N-(piperidin-4-ylmethyl)cyclopropanamine;
(trans)-2-(4-(benzyloxy)phenyl)-N-((1-methylpiperidin-4-yl)methyl)cyclopropanamine;
4-((4-((((1R,2S)-2-phenylcyclopropyl)amino)methyl)piperidin-1-yl)methyl)benzoic acid (GSK2879552);
1-((4-(methoxymethyl)-4-(((1R,2S)-2-phenylcyclopropylamino)methyl)piperidin-1-yl)methyl)cyclobutanecarboxylic acid;
N-[(2S)-5-{[(1R,2S)-2-(4-fluorophenyl)cyclopropyl]amino}-1-(4-methylpiperazin-1-yl)-1-oxopentan-2-yl]-4-(1H-1,2,3-triazol-1-yl)benzamide;
4-[2-(4-amino-piperidin-1-yl)-5-(3-fluoro-4-methoxy-phenyl)-1-methyl-6-oxo-1,6-dihydro-pyrimidin-4-yl]-2-fluorobenzonitrile; 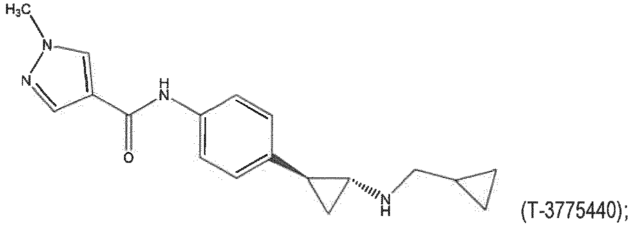 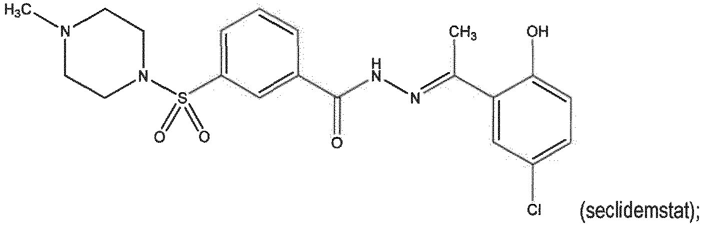 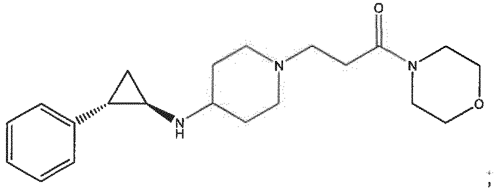 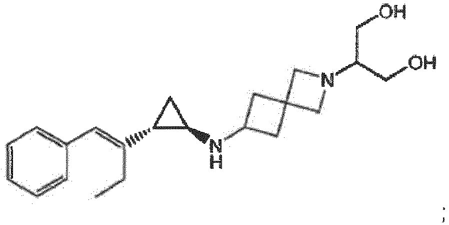 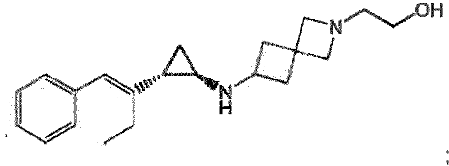
or 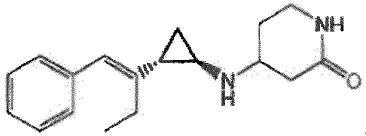
including any optically active stereoisomer thereof,
or a pharmaceutically acceptable salt or solvate thereof.

### Pharmaceutical Formulations

While it is possible that a KDM1A inhibitor, for example vafidemstat, may be administered for use in therapy directly as such, it is typically administered in the form of a pharmaceutical composition, which comprises the compound as active pharmaceutical ingredient together with one or more pharmaceutically acceptable excipients or carriers.

The KDM1A inhibitor may be administered by any means that accomplish the intended purpose. Examples include administration by the oral, parenteral (including e.g. intravenous, subcutaneous or intracerebral), or topical routes.

For oral delivery, the compound can be incorporated into a formulation that includes pharmaceutically acceptable carriers such as binders (*e.g.,* gelatin, cellulose, gum tragacanth), excipients (*e.g*., starch, lactose), lubricants (*e.g*., magnesium stearate, silicon dioxide), disintegrating agents (*e.g.,* alginate, Primogel, and corn starch), and sweetening or flavoring agents (*e.g*., glucose, sucrose, saccharin, methyl salicylate, and peppermint). The formulation can be orally delivered, e.g., in the form of enclosed gelatin capsules or compressed tablets. Capsules and tablets can be prepared by any conventional techniques. The capsules and tablets can also be coated with various coatings known in the art to modify the flavors, tastes, colors, and shapes of the capsules and tablets. In addition, liquid carriers such as fatty oil can also be included in capsules. Suitable oral formulations can also be in the form of suspension, syrup, chewing gum, wafer, elixir, and the like. If desired, conventional agents for modifying flavors, tastes, colors, and shapes of the special forms can also be included. In addition, for convenient administration by enteral feeding tube in patients unable to swallow, the active compounds can be dissolved in an acceptable lipophilic vegetable oil vehicle such as olive oil, corn oil and safflower oil.

The compound can also be administered parenterally in the form of solution or suspension, or in lyophilized form capable of conversion into a solution or suspension form before use. In such formulations, diluents or pharmaceutically acceptable carriers such as sterile water and physiological saline buffer can be used. Other conventional solvents, pH buffers, stabilizers, anti-bacteria agents, surfactants, and antioxidants can all be included. For example, useful components include sodium chloride, acetates, citrates or phosphates buffers, glycerin, dextrose, fixed oils, methyl parabens, polyethylene glycol, propylene glycol, sodium bisulfate, benzyl alcohol, ascorbic acid, and the like. The parenteral formulations can be stored in any conventional containers such as vials and ampoules.

For topical administration, the compound can be formulated into lotions, creams, ointments, gels, powders, pastes, sprays, suspensions, drops and aerosols. Thus, one or more thickening agents, humectants, and stabilizing agents can be included in the formulations. Examples of such agents include, but are not limited to, polyethylene glycol, sorbitol, xanthan gum, petrolatum, beeswax, or mineral oil, lanolin, squalene, and the like. A special form of topical administration is delivery by a transdermal patch. Methods for preparing transdermal patches are disclosed, *e.g*., in Brown, et al. (1988) Ann. Rev. Med. 39:221-229*.*

Subcutaneous implantation for sustained release of the compound may also be a suitable route of administration. This entails surgical procedures for implanting an active compound in any suitable formulation into a subcutaneous space, *e.g.,* beneath the anterior abdominal wall. See, *e.g*., Wilson et al. (1984) J. Clin. Psych. 45:242-247. Hydrogels can be used as a carrier for the sustained release of active compounds. Hydrogels are generally known in the art. They are typically made by crosslinking high molecular weight biocompatible polymers into a network, which swells in water to form a gel like material. Preferably, hydrogels are biodegradable or biosorbable. For purposes of this invention, hydrogels made of polyethylene glycols, collagen, or poly(glycolic-co-L-lactic acid) may be useful. See, *e.g*., Phillips et al. (1984) J. Pharmaceut. Sci., 73: 1718-1720.

Controlled release of an active compound can also be achieved by incorporating the active ingredient into microcapsules, nanocapsules, or hydrogels generally known in the art.

Liposomes can also be used as carriers for the active compound. Liposomes are micelles made of various lipids such as cholesterol, phospholipids, fatty acids, and derivatives thereof. Various modified lipids can also be used. Liposomes can reduce the toxicity of the active compounds, and increase their stability. Methods for preparing liposomal suspensions containing active ingredients therein are generally known in the art. See, e.g., U.S. Patent No. 4,522,811; Prescott, Ed., Methods in Cell Biology, Volume XIV, Academic Press, New York, N. Y. (1976).

The pharmaceutical compositions, like oral and parenteral compositions, can be formulated in unit dosage forms for ease of administration and uniformity of dosage. As used herein, "unit dosage forms" refers to physically discrete units suitable as unitary dosages for administration to subjects, each unit containing a predetermined quantity of active ingredient calculated to produce the desired therapeutic effect, in association with one or more suitable pharmaceutical carriers.

In therapeutic applications, pharmaceutical compositions are to be administered in a manner appropriate to the disease to be treated, as determined by a person skilled in the medical arts. An appropriate dose and suitable duration and frequency of administration will be determined by such factors as the condition of the patient, the type and severity of the disease, the particular form of the active ingredient, the method of administration, among others. In general, an appropriate dose and administration regimen provides the pharmaceutical composition in an amount sufficient to provide therapeutic benefit, for example an improved clinical outcome, such as more frequent complete or partial remissions, or longer disease-free and/or overall survival, or lessening of symptoms severity, or any other objetively identifiable improvement as noted by the clinician. Effective doses may generally be assessed or extrapolated using experimental models like dose-response curves derived from in vitro or animal model test systems, or from clinical trials.

The pharmaceutical compositions of the invention can be included in a container, pack or dispenser together with instructions for administration.

KDM1A inhibitors, such as vafidemstat, have been found to be orally active and to be effective in the treatment of ADHD when administered orally, as illustrated in Example 3. Accordingly, it is preferred that the KDM1A inhibitor (e.g., vafidemstat) is administered by the oral route for the treatment of ADHD.

The present invention also embraces the use of KDM1A inhibitors, in which one or more atoms are replaced by a specific isotope of the corresponding atom. For example, the invention encompasses the use of a KDM1A inhibitor, in which one or more hydrogen atoms (or, e.g., all hydrogen atoms) are replaced by deuterium atoms (i.e., ²H; also referred to as "D"). Accordingly, the invention also embraces KDM1A inhibitors which are enriched in deuterium. Naturally occurring hydrogen is an isotopic mixture comprising about 99.98 mol-% hydrogen-1 (¹H) and about 0.0156 mol-% deuterium (²H or D). The content of deuterium in one or more hydrogen positions in a KDM1A inhibitor can be increased using deuteration techniques known in the art. For example, a KDM1A inhibitor or a reactant or precursor to be used in the synthesis of the KDM1A inhibitor can be subjected to an H/D exchange reaction using, e.g., heavy water (D₂O). Further suitable deuteration techniques are described in: Atzrodt J et al., Bioorg Med Chem, 20(18), 5658-5667, 2012; William JS et al., Journal of Labelled Compounds and Radiopharmaceuticals, 53(11-12), 635-644, 2010; Modvig A et al., J Org Chem, 79, 5861-5868, 2014. The content of deuterium can be determined, e.g., using mass spectrometry or NMR spectroscopy. Unless specifically indicated otherwise, it is preferred that the KDM1A inhibitor to be used in accordance with the present invention is not enriched in deuterium. Accordingly, the presence of naturally occurring hydrogen atoms or ¹H hydrogen atoms in the KDM1A inhibitor is preferred. In general, it is preferred that none of the atoms in the KDM1A inhibitor to be used in accordance with the invention are replaced by specific isotopes.

### Definitions

Unless defined otherwise, all technical and scientific terms used herein have the same meaning as commonly understood by one of ordinary skill in the art to which this invention pertains.

The following definitions apply throughout the present specification and claims, unless specifically indicated otherwise.

A "patient" or "subject" for the purposes of the present invention includes both humans and other animals, particularly mammals. Thus, the methods and uses of the invention are applicable to both human therapy and veterinary applications. In a preferred aspect the subject or patient is a mammal, and in the most preferred aspect the subject or patient is a human (e.g. a male or female human).

The terms "treatment", "treating" and the like are used herein to generally mean obtaining a desired pharmacological and/or physiological effect. The effect may be prophylactic in terms of completely or partially preventing a disease (herein, ADHD) or symptom thereof and/or may be therapeutic in terms of partially or completely curing or ameliorating a disease (i.e. ADHD) and/or a symptom or adverse effect attributed to the disease or partially or completely halting the progression of a disease and/or a symptom or adverse effect attributed to the disease. The term "treatment" as used herein covers any treatment of a disease (i.e. ADHD) in a patient and includes, without limitation, any one or more of the following: (a) preventing ADHD in a patient which may be predisposed/at risk of developing ADHD; (b) delaying the onset of ADHD; (c) inhibiting ADHD, i.e. arresting, delaying or slowing down its development/progression; or (d) relieving the ADHD, i.e. causing (complete or partial) regression, correction or alleviation of ADHD.

As used herein, the term "therapeutically effective amount" refers to the amount sufficient to produce a desired biological effect (e.g., a therapeutic effect) in a subject. Accordingly, a therapeutically effective amount of a compound may be an amount which is sufficient to treat a disease (i.e. ADHD), and/or delay the onset or progression of the disease, and/or alleviate one or more symptoms of the disease, when administered to a subject suffering from or susceptible to that disease.

As used herein, the abbreviation "ADHD" refers to attention deficit hyperactivity disorder.

As used herein, a "pharmaceutically acceptable salt" is intended to mean a salt that retains the biological effectiveness of the free acids and/or bases of the specified compound and that is not biologically or otherwise undesirable. A compound may possess a sufficiently acidic, a sufficiently basic, or both functional groups, and accordingly react with any of a number of inorganic or organic bases, and inorganic and organic acids, to form a pharmaceutically acceptable salt. Exemplary pharmaceutically acceptable salts include those salts prepared by reaction of a compound according to the invention, e.g. vafidemstat with a mineral or organic acid, such as hydrochlorides, hydrobromides, sulfates, pyrosulfates, bisulfates, sulfites, bisulfites, phosphates, monohydrophosphates, dihydrophosphates, metaphosphates, pyrophosphates, chlorides, bromides, iodides, nitrates, acetates, propionates, decanoates, caprylates, acrylates, formates, isobutyrates, caproates, heptanoates, propiolates, oxalates, malonates, succinates, suberates, sebacates, fumarates, maleates, butyne-1,4-dioates, hexyne-1,6-dioates, benzoates, chlorobenzoates, methylbenzoates, dinitrobenzoates, hydroxybenzoates, methoxybenzoates, phthalates, sulfonates, xylenesulfonates, phenylacetates, phenylpropionates, phenylbutyrates, citrates, lactates, gamma-hydroxybutyrates, glycollates, tartrates, methane-sulfonates, ethane-sulfonates, propanesulfonates, benzenesulfonates, toluenesulfonates, trifluoromethansulfonates, naphthalene-1-sulfonates, naphthalene-2-sulfonates, mandelates, pyruvates, stearates, ascorbates, or salicylates. When a compound carries an acidic moiety, suitable pharmaceutically acceptable salts thereof may include alkali metal salts, e.g. sodium or potassium salts; alkaline earth metal salts, e.g. calcium or magnesium salts; and salts formed with suitable organic ligands such as ammonia, alkylamines, hydroxyalkylamines, lysine, arginine, N-methylglucamine, procaine and the like. Pharmaceutically acceptable salts are well known in the art.

As used herein, a "pharmaceutically acceptable solvate" refers to a complex of variable stoichiometry formed by a solute and a pharmaceutically acceptable solvent such as water, ethanol and the like. A complex with water is known as a hydrate. It is to be understood that the invention encompasses pharmaceutically acceptable solvates of vafidemstat in non-salt form and also in the form of a pharmaceutically acceptable salt thereof.

As used herein, a "pharmaceutically acceptable carrier" or "pharmaceutically acceptable excipient" refers to non-API (API refers to Active Pharmaceutical Ingredient) substances such as disintegrators, binders, fillers, and lubricants used in formulating pharmaceutical products. They are generally safe for administering to humans according to established governmental standards, including those promulgated by the United States Food and Drug Administration and/or the European Medicines Agency. Pharmaceutically acceptable carriers or excipients are well known to those skilled in the art.

As used herein, a "small molecule" refers to an organic compound with a molecular weight below 900 daltons, preferably below 500 daltons. The molecular weight is the mass of a molecule and is calculated as the sum of the atomic weights of each constituent element multiplied by the number of atoms of that element in the molecular formula.

As used herein, the term "comprising" (or "comprise", "comprises", "contain", "contains", or "containing"), unless explicitly indicated otherwise or contradicted by context, has the meaning of "containing, inter alia", i.e., "containing, among further optional elements, ...". In addition thereto, this term also includes the narrower meanings of "consisting essentially of' and "consisting of". For example, the term "A comprising B and C" has the meaning of "A containing, inter alia, B and C", wherein A may contain further optional elements (e.g., "A containing B, C and D" would also be encompassed), but this term also includes the meaning of "A consisting essentially of B and C" and the meaning of "A consisting of B and C" (i.e., no other components than B and C are comprised in A).

As used herein, unless explicitly indicated otherwise or contradicted by context, the terms "a", "an" and "the" are used interchangeably with "one or more" and "at least one". Thus, for example, a composition comprising "a" KDM1A inhibitor can be interpreted as referring to a composition comprising "one or more" KDM1A inhibitors.

### EXAMPLES

The following examples illustrate various aspects of the invention. The examples should, of course, be understood to be merely illustrative of only certain embodiments of the invention. Results are also presented and described in the Figures and Figure legends.

### Example 1: KDM1A inhibitor

Vafidemstat is the compound 5-((((1R,2S)-2-(4-(benzyloxy)phenyl)cyclopropyl)amino)methyl)-1,3,4-oxadiazol-2-amine, also known as (-) 5-((((trans)-2-(4-(benzyloxy)phenyl)cyclopropyl)amino)methyl)-1,3,4-oxadiazol-2-amine, (41R,42S)-6-oxa-3-aza-1(2)-[1,3,4]oxadiazola-5(1,4),8(1)-dibenzena-4(1,2)-cyclopropanaoctaphan-15-amine or ORY-2001, and whose chemical structure is shown below.

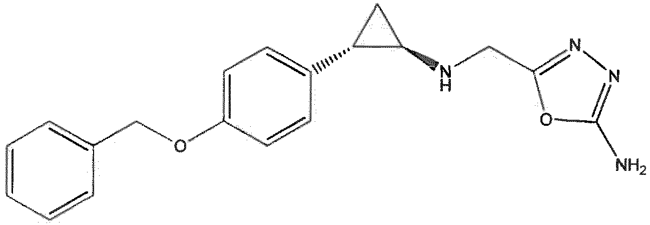

This compound can be obtained as disclosed in WO2012/013728.

### Example 2: In vitro KDM1A inhibition assay

The inhibitory activity of a compound against KDM1A can be determined using the method described below. Human recombinant KDM1A protein (GenBank accession no. NM_015013, amino acids 158-end with N-terminal GST tag, MW: 103 kDa) was used.

Serial 3-fold dilutions of a test compound ranged between 30 µM and 1 nM were pre-incubated for 15 min with human recombinant KDM1A enzyme (BPS Bioscience, Ref. 50100) on ice in the assay buffer (50 mM sodium phosphate pH 7.4). Each concentration of inhibitor was tested in duplicate. The enzymatic reaction was initiated by the addition of dimethyl H3K4 peptide substrate (Anaspec, Ref. 63677), at the appK_{M} of KDM1A. After 30 min of incubation at 37°C Amplex Red reagent and the horseradish peroxidase (HRP) solution were added to detect H₂O₂ formed in the enzymatic reaction, following the recommendations provided by the supplier (Invitrogen). The mix was incubated for 5 min at room temperature in the dark and the conversion of the Amplex Red reagent to the highly fluorescent resorufin was analyzed using an Infinite F200 Tecan fluorescence microplate reader (λexcitation=540 nm, λemission=590 nm). The maximum demethylase activity of KDM1A was obtained in the absence of inhibitor and corrected for background fluorescence in the absence of KDM1A. The IC₅₀ value for each inhibitor was calculated with GraphPad Prism5 Software from a minimum of two independent experiments.

Vafidemstat is a KDM1A inhibitor, as shown by a mean IC₅₀ value of 101 ± 40 nM obtained in the KDM1A assay described herein.

### Example 3: Evaluation of the effect of KDM1A inhibitors to treat ADHD in humans

As part of a Phase IIa clinical trial (REIMAGINE trial, EudraCT number 2018-002140-88) to evaluate the safety, tolerability and efficacy of the KDM1A inhibitor vafidemstat to treat aggression in adult population in patients with different CNS disorders, a cohort of ADHD patients was recruited and treated with vafidemstat for 8 weeks. A summary of the protocol of this clinical trial and results obtained in the ADHD cohort are provided below.

### 3.1 Clinical trial design

Reimagine is a unicenter, open-label, 1-arm, 8-week clinical study to evaluate the efficacy, safety and tolerability of vafidemstat in aggression in adult population with Alzheimer's Disease (AD), Lewy Body Dementia (LBD), Adult attention Deficit Hyperactivity Disorder (ADHD), Borderline Personality Disorder (BPD) and Autism Spectrum Disorder (ASD). Six patients to be recruited per disorder.

Main objective of the trial: To evaluate the safety and tolerability of vafidemstat in adult population with Alzheimer's Disease (AD), Lewy Body Dementia (LBD), Adult attention deficit hyperactivity disorder (ADHD), Borderline Personality Disorder (BPD), Autism Spectrum Disorder (ASD)

Secondary objectives of the trial: To investigate the efficacy of vafidemstat in aggression in adult population with Alzheimer's Disease (AD), Lewy Body Dementia (LBD), Adult attention deficit hyperactivity disorder (ADHD), Borderline Personality Disorder (BPD), Autism Spectrum Disorder (ASD)

Main inclusion criteria:
- age 18-85
- current diagnosis for AD, LBD, ADHD, BPD or ASD according to DSM-5 criteria
- significant or persistent agitation or aggression that was disruptive to patient's daily living or put the patient in harm's way for at least 3 days per week for at least 4 weeks prior to screening visit

Treatment: All patients received vafidemstat (as free base) at a dose of 1.2 mg/day, administered orally as a single capsule, in a 5 days on/2 days off schedule, during 8 weeks.

### 3.2 ADHD cohort

Six ADHD patients were recruited, but there was one drop-out, and therefore the results as described herein correspond to the 5 ADHD subjects eligible for analysis. A summary of the patients recruited in this ADHD cohort (demographic data at baseline) can be found in Table 1.

**Table 1:**

| **Demographic data ADHD patients** | | |
|---|---|---|
| n° of patients | 6 | |
| Sex | Male | 5 (83.3 %) |
| | Female | 1(16.6 %) |
| Age | Median (years) | 36,00 |
| | (Min , Max ) | (19/53) |
| Race | Caucasian | 4(66.6 %) |
| | Other | 2(33.3 %) |
| Weight | Median (Kg) | 73.17 |
| | (Min , Max ) | 62/101 |
| Height | Median (cm) | 169.50 |
| | (Min , Max ) | (160/177) |
| BMI | Median | 25.49 |
| | (Min , Max ) | (19.15/36.07) |

### 3.3 Efficacy assessments in the ADHD cohort

Evaluation of the effect of treatment with vafidemstat on aggression was performed using the Clinical Global Impression (CGI) scale. CGI values reflect clinician's ratings of patients' aggression severity (CGI-S scale) and improvement in aggression from the initiation (baseline) of the treatment (CGI-I scale). In the CGI-S scale, illness/condition severity is rated using a seven-point scale, the higher the score, the more severe the illness/condition, with 1 = normal, not at all ill and 7 = among the most extremely ill patients. In the CGI-I scale, change from initiation of treatment is rated also using a seven-point scale, where 1 = very much improved since initiation of treatment, 7= very much worse since initiation of treatment, and 4 = no change from baseline.

In addition to assessing its effect on aggression, the effect of treatment with vafidemstat on ADHD patients was also evaluated using the Attention Deficit Hyperactivity Disorder Rating Scale (ADHD-RS), a validated scale that is specific for ADHD and is widely used to evaluate ADHD in adults. The ADHD-RS is a scale of 18 items reflecting DSM-5 criteria for ADHD, and consists of a lack of attention sub-scale (9 items) and a hyperactivity/impulsiveness subscale (9 items). Each item is scored from 0 to 3 points, with the highest scores indicating the presence of problematic symptoms/behavior. None of the 18 items included in the ADHD-RS scale are directly related or specifically aimed to the evaluation of aggressive behavior. Therefore, the ADHD-RS scale may be used to assess the effects of treatment with vafidemstat on ADHD independently of the evaluation of effects of this drug on aggression.

All efficacy evaluations were performed by assessing the change from baseline (visit 1) to week 8 (visit 7) of the respective scale scores (CGI-S, CGI-I and ADHD-RS).

Statistical analysis was performed using paired one-tail t-test analysis to compare Visit 1 with Visit 7 values.

### 3.4 Results

Treatment with vafidemstat in ADHD patients was safe and well tolerated, without significant adverse events. Treatment of ADHD patients with vafidemstat for 8 weeks produced a significant improvement in aggression, as shown by statistically significant reductions of the CGI-S and CGI-I values from visit 1 to visit 7, as depicted in Figures 1A (CGI-S, p= 0.0043) and 1B (CGI-I, p= 0.0352).

Unexpectedly, in addition to producing an improvement in aggression, the ADHD-RS score showed a statistically significant reduction after 2 months of treatment with vafidemstat, as shown in Figure 2 (p=0.0279).

As the ADHD-RS scale does not include any items focusing on aggression, as discussed above, the effects observed in the ADHD-RS scale show that KDM1A inhibitors such as vafidemstat exert therapeutic effects in ADHD patients beyond a therapeutic effect on aggression.

In summary, the data and results provided in Example 3 support the use of KDM1A inhibitors, particularly vafidemstat, for the treatment of ADHD, including the treatment of ADHD core features or ADHD symptoms unrelated to aggression.

Using the protocol described herein in Example 3, the therapeutic effects of other KDM1A inhibitors as a treatment for ADHD can be verified.

The publications, patents and patent applications mentioned in the specification are provided solely for their disclosure prior to the filing date of the present application. Nothing herein is to be construed as an admission that they are prior art to the instant application.

## Claims

1. A KDM1A inhibitor for use in the treatment of attention deficit hyperactivity disorder (ADHD) by treating one or more non-aggressive symptoms of ADHD, wherein the KDM1A inhibitor is 5-((((1R,2S)-2-(4-(benzyloxy)phenyl)cyclopropyl)amino)methyl)-1,3,4-oxadiazol-2-amine or a pharmaceutically acceptable salt or solvate thereof.

2. A pharmaceutical composition for use in the treatment of attention deficit hyperactivity disorder (ADHD) by treating one or more non-aggressive symptoms of ADHD, wherein the pharmaceutical composition comprises a KDM1A inhibitor and one or more pharmaceutically acceptable excipients or carriers, wherein the KDM1A inhibitor is 5-((((1R,2S)-2-(4-(benzyloxy)phenyl)cyclopropyl)amino)methyl)-1,3,4-oxadiazol-2-amine or a pharmaceutically acceptable salt or solvate thereof.

3. The compound for use according to claim 1 or the pharmaceutical composition for use according to claim 2, wherein the patient to be treated is a human.

4. The compound for use according to claim 1 or 3 or the pharmaceutical composition for use according to claim 2 or 3, wherein the KDM1A inhibitor is 5-((((1R,2S)-2-(4-(benzyloxy)phenyl)cyclopropyl)amino)methyl)-1,3,4-oxadiazol-2-amine.

5. The compound for use according to any one of claims 1, 3 or 4 or the pharmaceutical composition for use according to any one of claims 2 to 4, wherein the KDM1A inhibitor or the pharmaceutical composition is administered orally.

6. The compound for use according to any one of claims 1 or 3 to 5 or the pharmaceutical composition for use according to any one of claims 2 to 5, wherein the attention deficit hyperactivity disorder is adult attention deficit hyperactivity disorder.

## Patentansprüche

1. Ein KDM1A-Inhibitor zur Verwendung bei der Behandlung von Aufmerksamkeitsdefizit-/Hyperaktivitätsstörung (ADHS) durch Behandeln eines oder mehrerer nicht-aggressiver Symptome von ADHS, wobei der KDM1A-Inhibitor 5-((((1R,2S)-2-(4-(Benzyloxy)phenyl)cyclopropyl)amino)methyl)-1,3,4-oxadiazol-2-amin oder ein pharmazeutisch verträgliches Salz oder Solvat davon ist.

2. Eine pharmazeutische Zusammensetzung zur Verwendung bei der Behandlung von Aufmerksamkeitsdefizit-/Hyperaktivitätsstörung (ADHS) durch Behandeln eines oder mehrerer nicht-aggressiver Symptome von ADHS, wobei die pharmazeutische Zusammensetzung einen KDM1A-Inhibitor und einen oder mehrere pharmazeutisch verträgliche Exzipienten oder Träger umfasst, wobei der KDM1A-Inhibitor 5-((((1R,2S)-2-(4-(Benzyloxy)phenyl)cyclopropyl)amino)methyl)-1,3,4-oxadiazol-2-amin oder ein pharmazeutisch verträgliches Salz oder Solvat davon ist.

3. Die Verbindung zur Verwendung gemäß Anspruch 1 oder die pharmazeutische Zusammensetzung zur Verwendung gemäß Anspruch 2, wobei der zu behandelnde Patient ein Mensch ist.

4. Die Verbindung zur Verwendung gemäß Anspruch 1 oder 3 oder die pharmazeutische Zusammensetzung zur Verwendung gemäß Anspruch 2 oder 3, wobei der KDM1A-Inhibitor 5-((((1R,2S)-2-(4-(Benzyloxy)phenyl)cyclopropyl)amino)methyl)-1,3,4-oxadiazol-2-amin ist.

5. Die Verbindung zur Verwendung gemäß einem der Ansprüche 1, 3 oder 4 oder die pharmazeutische Zusammensetzung zur Verwendung gemäß einem der Ansprüche 2 bis 4, wobei der KDM1A-Inhibitor oder die pharmazeutische Zusammensetzung oral verabreicht wird.

6. Die Verbindung zur Verwendung gemäß einem der Ansprüche 1 oder 3 bis 5 oder die pharmazeutische Zusammensetzung zur Verwendung gemäß einem der Ansprüche 2 bis 5, wobei die Aufmerksamkeitsdefizit-/Hyperaktivitätsstörung eine Aufmerksamkeitsdefizit-/Hyperaktivitätsstörung bei Erwachsenen ist.

## Revendications

1. Inhibiteur de KDM1A pour une utilisation dans le traitement du trouble du déficit de l'attention avec hyperactivité (TDAH) par traitement d'un ou plusieurs symptômes non agressifs du TDAH, lequel inhibiteur de KDM1A est la 5-((((1R,2S)-2-(4-(benzyloxy)phényl)cyclopropyl)amino)méthyl)-1,3,4-oxadiazol-2-amine ou un solvate ou sel pharmaceutiquement acceptable de celle-ci.

2. Composition pharmaceutique pour une utilisation dans le traitement du trouble du déficit de l'attention avec hyperactivité (TDAH) par traitement d'un ou plusieurs symptômes non agressifs du TDAH, laquelle composition pharmaceutique comprend un inhibiteur de KDM1A et un ou plusieurs véhicules ou excipients pharmaceutiquement acceptables, dans laquelle l'inhibiteur de KDM1A est la 5-((((1R,2S)-2-(4-(benzyloxy)phényl)cyclopropyl)amino)méthyl)-1,3,4-oxadiazol-2-amine ou un solvate ou sel pharmaceutiquement acceptable de celle-ci.

3. Composé pour une utilisation selon la revendication 1 ou composition pharmaceutique pour une utilisation selon la revendication 2, dans lequel le patient devant être traité est un humain.

4. Composé pour une utilisation selon la revendication 1 ou 3 ou composition pharmaceutique pour une utilisation selon la revendication 2 ou 3, dans lequel l'inhibiteur de KDM1A est la 5-((((1R,2S)-2-(4-(benzyloxy)phényl)cyclopropyl)amino)méthyl)-1,3,4-oxadiazol-2-amine.

5. Composé pour une utilisation selon l'une quelconque des revendications 1, 3 et 4 ou composition pharmaceutique pour une utilisation selon l'une quelconque des revendications 2 à 4, dans lequel l'inhibiteur de KDM1A ou la composition pharmaceutique est administré par voie orale.

6. Composé pour une utilisation selon l'une quelconque des revendications 1 et 3 à 5 ou composition pharmaceutique pour une utilisation selon l'une quelconque des revendications 2 à 5, dans lequel le trouble du déficit de l'attention avec hyperactivité est un trouble du déficit de l'attention avec hyperactivité de l'adulte.
